# EUROPEAN PATENT SPECIFICATION

(11) **EP 2 046 646 B1**
(45) Date of publication and mention of the grant of the patent: **30.11.2011**
(21) Application number: 07789601.7
(22) Date of filing: 02.08.2007
(51) Int. Cl.: B65B 3/16, B65B 3/32, B65D 35/22, B65D 77/08

(54) **METHOD AND EQUIPMENT FOR FILLING PROGRESSIVE EFFECT MULTI-PHASE PRODUCTS, AS WELL AS THE PRODUCTS THEMSELVES**
VERFAHREN UND EINRICHTUNG ZUM FÜLLEN VON MEHRPHASENPRODUKTEN MIT PROGRESSIVER WIRKUNG SOWIE DIE PRODUKTE SELBST
PRODUITS À PHASES MULTIPLES À EFFET PROGRESSIF, PROCÉDÉ DE REMPLISSAGE DE CEUX-CI ET ÉQUIPEMENT POUR METTRE EN OEUVRE LE PROCÉDÉ

(30) Priority: 04.08.2006 IT MI20061578
(43) Date of publication of application: 15.04.2009
(73) Proprietor: Safosa S.p.a., 20083 Gaggiano (MI) (IT)
(72) Inventor: CASERIO, Domenico, 20127 Milano (IT)
(74) Representative: Mati, Silvia
(86) International application number: PCT/IB2007/002227
(87) International publication number: WO 2008/015556

(56) References cited:
- EP-A- 0 243 321
- EP-A- 1 657 159
- DE-A1- 3 503 058
- DE-A1- 19 735 619
- US-A- 1 760 773
- US-A- 4 254 894

## Description

The present invention refers to multi-phase products having a progressive effect, to a filling method thereof and to an equipment for carrying out such a method.

To date, cream, paste or gel products, such as toothpastes, are already known, which consist of two or more different product phases, accommodated in a suitable container.

Such product phases sometimes comprise different active ingredients, such as an ingredient with a refreshing action and an ingredient with a whitening action, and sometimes they are only visually differentiated in that they generally exhibit a different colour or a different formulation, such as a paste, a cream or a gel.

To this end, the individual product phases have suitable plasticity and viscosity characteristics that prevent them from mixing and they comprise suitable ingredients and dyes that hinder the transfer of colours, active ingredients and so on, from one phase to the other.

In order to obtain these products, methods for filling containers are adopted today, in which two or more product phases are placed longitudinally with respect to each other along the main extension of the containers.

With such methods it is possible to obtain a multi-phase product, which always displays the same phase composition throughout the entire lifetime of the product.

Such filling methods are employed in particular in the toothpaste field and generate products that are distinguished by the arrangement of their individual phases within the container.

The product designated as "deep stripe", for example, presents phases that are longitudinally and substantially circularly arranged one alongside the other. The product dispensed in the form of a cylindrical portion therefore has a plurality of sections, each formed by a product phase.

Similarly, the so called "superficial stripe" has a first phase accommodated in a plurality of adjacent portions of the container opening. Such first phase is dispensed from the container by entrainment of a second phase accommodated in the remainder of the container body, thus forming several stripes on the surface of the second phase. The so called "coex" product instead has two phases arranged one alongside the other that are dispensed by coextrusion.

Finally, the "included concentric coex" product has a first phase located inside and substantially at the centre of a second phase, so that the product phases are longitudinally concentric with respect to each other as they are dispensed.

As a result, with the filling methods heretofore known, it is only possible to obtain products which, as they are dispensed from their containers, have a composition of longitudinally mutually separated phases having an arrangement and composition essentially always equal for the entire duration of dispensing product.

The amount of dispensed product being equal, the proportion of the product phases dispensed, and accordingly of the concentrations of active ingredients available for use, remains constant throughout the entire lifetime of the product.

Therefore, with known multi-phase products it is not possible to obtain - given a specific dispensed amount - a supply in which the amount of one phase can be progressively increased and/or decreased with respect to the other phases making up the product, and accordingly it is not possible to achieve a progressive effect by dispensing different amounts of active ingredients.

The task of the present invention is to solve the above-mentioned drawbacks in multi-phase products of known types, by devising a product which can provide a substantially different functional and/or sensory effect upon each supply.

In the context of this task, an aim of the present invention is to realize a multi-phase product wherein the individual phases can be dispensed in a progressively increasing and/or decreasing amount.

A further aim of the present invention is to devise a multi-phase product which can make available for use a different combination of active ingredients upon each supply.

Another aim of the present invention is to devise a filling method of a multi-phase product that allows the individual phases to be dispensed in a progressively increasing and/or decreasing amount.

The last but not least aim of the present invention is to perfect a filling equipment so that it can implement such method.

This task and other aims according to the present invention are achieved by a multi-phase product as recited in claim 1.

Similarly, the aims according to the present invention are achieved by the filling method according to claim 11 or 12 and by the filling equipment defined in claim 18. Further characteristics of the invention are set forth in the remaining claims.

Such a dispensing with a progressive effect can be applied in several fields, including cosmetics, pharmaceutics, food industry, plant care products, household products in general and so on.

In an exemplary manner, in the cosmetics field it is possible to apply the multi-phase product according to the present invention to a sunscreen lotion composed of two or more phases that differ from each other in that they provide a different degree of sun protection. A gradual supply of the different phases would permit to have the higher protection phase dispensed first, when is it assumed that a higher protection should be employed, and then lower protection phases dispensed in a progressive manner, until only one lowest protection phase is dispensed.

To this end, it is necessary to provide a cream with plasticity characteristics such that the individual phases can be accommodated in the same container without becoming mixed or uniform.

Such application offers the convenience of carrying along a single product having the possibility, however, to reduce progressively the sun protection adopted, for example with an increased tan. As regards toothpastes, it is possible to apply the multi-phase product according to the present invention to toothpaste that effects a stosstherapy for relieving gum inflammation or for teeth whitening, for example. In this case, a high initial quantity of active ingredient is required, the active ingredient being relieving or whitening respectively, that can be then gradually replaced by a second active ingredient for maintaining the effects obtained.

A product that can be dispensed in such a manner can also be extremely attractive in the market, in particular in the event the colours and/or flavours of the two phases are different from each other. A toothpaste which changes progressively in colour and/or flavour at each supply would be obtained. Similarly, in the pharmaceutics and parapharmaceutics field, it is possible to create products that can be employed for particular treatments, wherein a determined active ingredient has to be first delivered in high quantities and progressively replaced by a second ingredient.

In the food industry, it is possible to provide products, such as confectionery paste contained in tubes, that progressively change in flavour and/or colour with each supply, which generates market attractiveness in this case as well. Similarly, several other applications can be devised.

The characteristics and advantages of the present invention will result clearer from the description of certain preferred but not exclusive embodiments of a multi-phase product according to the invention, of the filling method thereof, as well as of the equipment employed to this end, which are illustrated in an illustrative but not limiting manner in the accompanying drawings, wherein:
- figures 1a-1d, respectively, are schematic representations in partial section of four embodiments of the multi-phase product according to the invention;
- figures 2a-2d illustrate several steps of the product filling method of figure 1a;

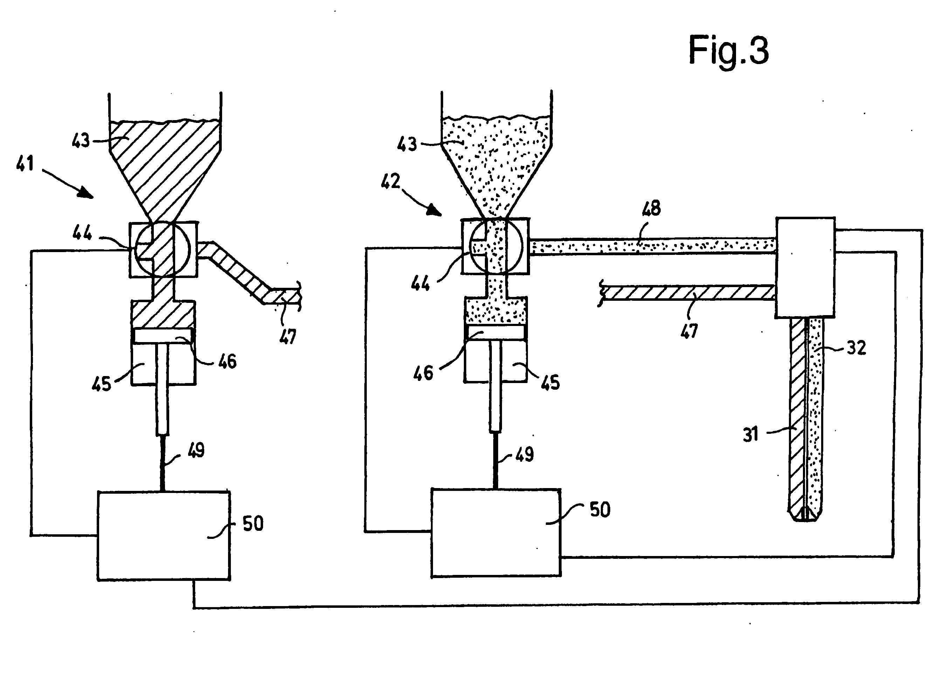

- figure 3 schematically shows the multi-phase product filling equipment with progressive supply of the single phases according to the invention;
- figure 4 is a graph illustrating the quantity of a dispensed active ingredient, contained in a first phase of the multi-phase product according to the invention versus number of supplies of a predetermined quantity. With reference to the cited figures, the multi-phase product with progressive supply of the single phases is generally denoted with 10.

The multi-phase product 10 comprises a container 11, wherein at least two product phases are accommodated 12a,12b, having physical characteristics that prevent their mutual mixing or a significant transfer of any dyes, active ingredients and the like from one phase to the other.

In order for the product phases 12a,12b to be dispensable, suitable containers 11 are employed, such as collapsible material tubes that allow partial discharge of phases 12a,12b following a proper pressure applied on the outside, or airless or screw dispensers that discharge phases 12a,12b by means of the movement of a piston within the container. In particular, phases 12a,12b are accommodated in the container 11 so that they form at least three container portions I, II, III that differ from each other in the composition of the phases 12a,12b therein contained.

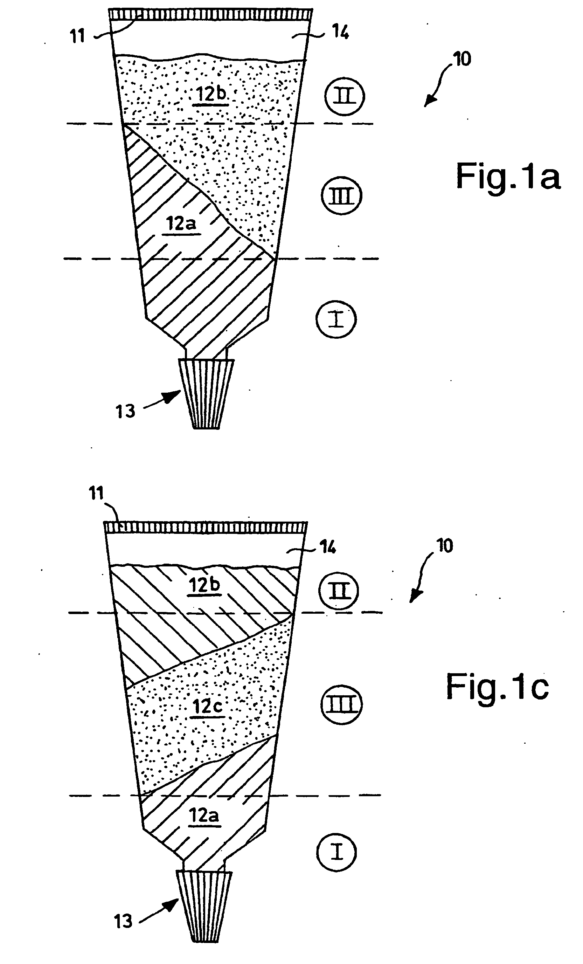

The particular embodiment of figure 1a features:
- a first container portion I close to a container opening 13 in which only one first phase 12a is accommodated;
- a second container portion II close to the bottom of the container and bordered on one side by residual air 14 in the container in which only one second product phase 12b is accommodated and the first product phase 12a is completely absent; and
- a third intermediate container portion III in which the two phases 12a,12b are longitudinally placed alongside each other in an oblique progression with respect to the main dimension of the container 11.

It is also possible to provide an inverted arrangement in which the first portion I is at the bottom of the container 11 and the second portion II is close to the opening 13 of the container 11.

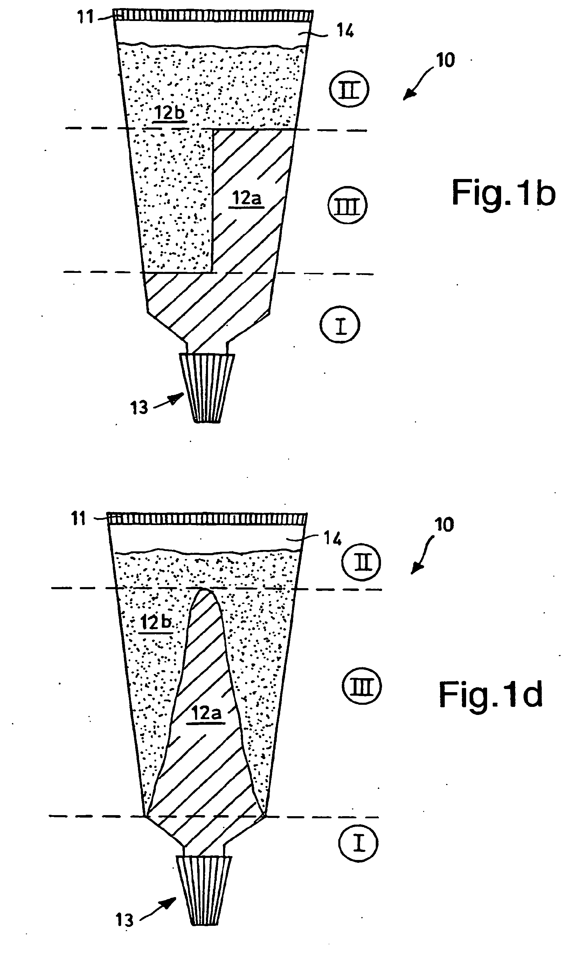

Similarly, in figure 1b, a second embodiment is shown, in which the intermediate portion III has two component phases 12a, 12b longitudinally placed alongside each other in a parallel direction with respect to the main dimension of the container 11.

In figure 1c a third embodiment is shown also having three container portions as described above. In particular, the intermediate portion III comprises three product phases 12a,12b,12c longitudinally placed alongside each other in an oblique manner.

Finally, in figure 1d a further embodiment is shown, wherein in the intermediate phase III the two phases 12a,12b are longitudinally brought together so that the first phase 12a forms a cone embedded in the second phase 12b.

With the products illustrated in figures 1a-1d and hereinabove described a progressive dispensing of the individual phases is obtained.

As regards the embodiments 1a, 1b, and 1d, in general only the first product phase 12a is dispensed first, followed by both phases 12a,12b being dispensed in a proportion that varies according to the total amount of product already dispensed.

In a similar manner, the embodiment of figure 1c also provides for the dispensing of an intermediate product phase 12c in a first increasing then decreasing manner.

During the dispensing step, wherein more product phases 12a,12b,12c flow out simultaneously, the relative amount of the first phase 12a decreases to zero and the last phase 12b increases, thereby ultimately replacing the other phases 12a,12c completely.

The decrease and corresponding increase of the relative amounts occur in a more or less gradual manner depending on the specific embodiment.

In order to confirm this, the Applicant carried out several laboratory tests resulting in a successive replacement of product phases 12a,12b,12c during the supply of the content of the multi-phase product 10 according to the invention.

To this end, the supply of multi-phase product 10 was divided into 49 sub-supplies of preset amount which were compared with each other.

It was found that the colour intensity and shade of the single (sub)supplies changed due to the use of product phases having different colours (white/green) and formulations (paste/gel). In particular, the colour changed progressively from the colour of the first product phase 12a to the colour of the last product phase 12b.

Moreover, from a chemical analysis of the individual supplies, the concentration of the particular chemical compound - sodium fluoride - only contained in the first product phase 12a, was found to decrease, from a maximum percentage corresponding to the percentage of active ingredient contained in the first product phase 12a, to substantially zero after mixing with further product phases 12b,12c lacking sodium fluoride.

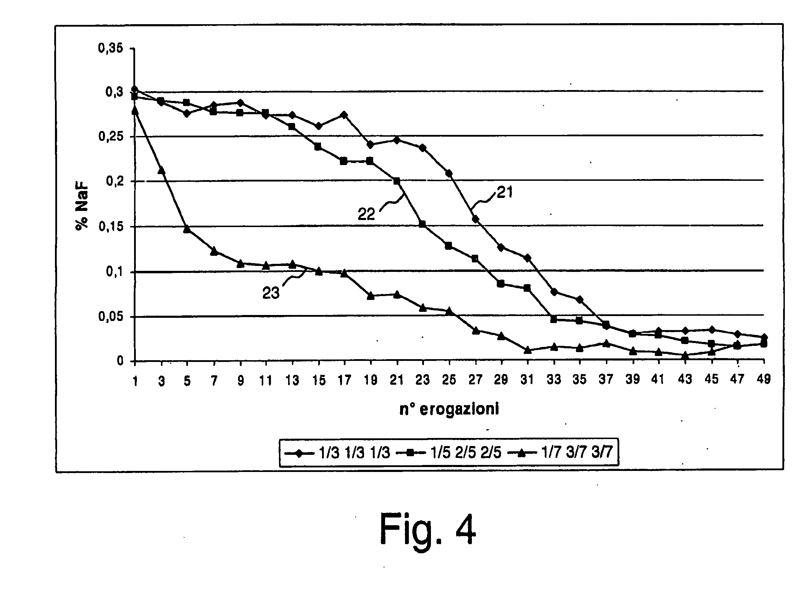

The above-mentioned results are reported as an example in the graph of figure 4.

Such analyses also demonstrated that the gradual progressive variation of the concentration of active ingredients contained in the particular product phases 12a,12b,12c depends on several factors including
- different types of container 11 filling (such as those in figures 1a-1d);
- relative proportions of the product phases employed;
- different filling ratios of the three portions I, II, III.

To substantiate this, three curves 21,22,23 are depicted in figure 4, each relating to a filling of the type in figure 1b, that differ both in the percent ratios of each one of the two product phases 12a,12b with respect to the total volume, and in the filling proportions of the three container 11 portions.

In particular, the first curve 21 relates to a filling in which each container portion I, II, III comprises one third of the total volume of product 10 and the proportion between the two phases is 50/50; the second curve 22 refers to a filling in which the proportion between the two phases is 40/60 distributed so that the first portion I, containing the first product phase 12a, is filled with one fifth of the total volume, while the remaining two portions II, III each contain two fifths of the total volume; and the third curve 23 relates to the case where the proportion between the two phases is 35.7/64.3 distributed so that the first portion I, containing the first product phase 12a, contains one seventh of the total volume, while the remaining two portions II,III are each filled with three seventh of the total volume.

However, the most varied proportions among the three container portions are possible.

From the results obtained, the most suitable filling proportions for obtaining the most gradual progression possible of the curve of figure 4 are:
- portion I: 1/5, portion II: 2/5, portion III: 2/5; and
- portion I: 1/4, portion II: 2/4, portion III: 1/4.

Furthermore, with only two product phases, the proportions between the two phases can range from 50/50 to 5/95 or 95/5 respectively, preferably from 50/50 to 10/90 or 90/10 respectively.

In order to obtain the multi-phase products 10 according to the invention, the Applicant devised an innovative filling method of containers 11.

Such method is characterised by effecting the filling of a container 11 in at least three steps by using a number of nozzles 31,32 corresponding to the number of product phases 12a,12b,12c making up the multi-phase product 10.

The single filling steps differ in how the opening and/or closing of the nozzles 31,32 is driven: in a first step only nozzle 31 is driven open, containing the product phase 12a which will fill the container 11 first portion I; during a second step at least two nozzles 31,32 are driven open simultaneously; and, in a third step the first product phase 12a nozzle 31 is driven closed:

The opening time of nozzles 31,32 is thus mutually offset.

In this manner, in the container part 11 close to the opening 13, there is a higher amount of a first product phase 12a that substantially covers the entire diameter of the tube, this amount decreasing as the filling of the container 11 increases, until it disappears in favour of the other product phases 12b,12c.

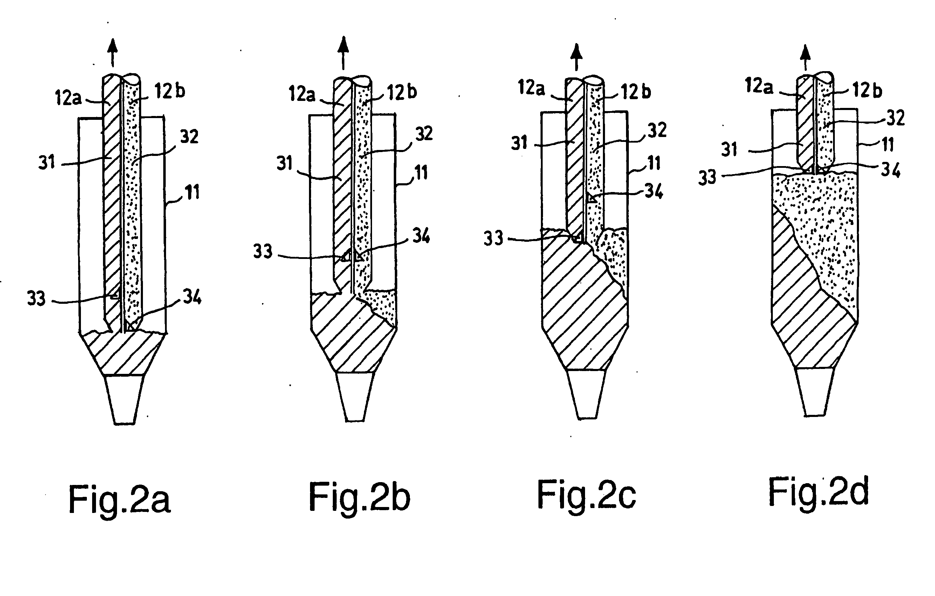

In particular, in order to obtain the multi-phase product 10 illustrated in figure 1a, the filling method according to the invention comprises the following steps:
a) the container 11 is brought to the filling station;
b) two nozzles 31,32, arranged side by side, are immersed in the container 11 until they reach its lower dead point;
c) the shutter 33 of a first nozzle 31 is opened and the metering of the first product phase 12a is started (see figure 2a);
d) after a first predefined offset, the shutter 34 of a second nozzle 32 is gradually opened, containing a second product phase 12b. During this step both product phases 12a,12b are simultaneously metered (see figures 2b and 2c);
e) after a second predefined offset, the shutter 33 of first nozzle 31 is gradually closed so that during this step only the second product phase 12b is metered (see figure 2d).

During the filling steps c) -e), nozzles 31,32 are gradually extracted from container 11.

In a like manner, in the event the number of product phases 12a,12b,12c is more than two, further steps can be provided in which corresponding nozzles are opened for offset periods with respect to the first product phase 12a feeding nozzle.

Also similarly, the sequence of steps can be reversed so that the first product phase 12a nozzle 31 remains initially closed, is opened and operates simultaneously with at least another nozzle 32 in a subsequent step, and stays opened alone in a final step.

In order to obtain different filling configurations such as those depicted in figures 1b-1d, it is possible to act upon the opening gaps of the nozzle, as well as on the opening and closing rate thereof.

Furthermore, the arrangement and the number of the nozzles can also influence the final geometry of the product phases 12a,12b,12c without affecting the basic principle of gradualness. For example, it is possible to provide a first nozzle 31 arranged within a second nozzle 32 so as to achieve the configuration illustrated in figure 1d.

In order to implement the described innovative method, the Applicant has perfected a filling equipment 40 consisting of at least two fully independent filling assemblies 41,42, each comprising a hopper 43 fed with a product phase 12a,12b,12c and driven open and/or closed by means of a rotating three-way valve 44. The filling assemblies 41,42 also comprise a metering cylinder 45, wherein a piston 46 is present that can slide inside the cylinder 45, and manifolds 47, 48 for connection to the respective nozzles 31,32.

Each filling assembly 41,42 is driven by an actuator means 50, such as a mechanical or electronic cam, like for example a brushless motor, simultaneously driving the piston 46 either downwards or upwards, and the three-way valve 44 from a connecting position between hopper 43 and cylinder 45 to a connecting position between cylinder 45 and the respective manifold 47,48.

In an inventive manner, for example, the mechanical cam 50 profiles of every assembly were defined so as to drive the corresponding filling assemblies 41,42 in a mutually offset manner, i.e. so that these filling assemblies 41,42 are not opened and/or closed simultaneously, but in a time offset manner with respect to each other.

The operation of the filling equipment is as follows.

At every machine cycle, the first filling assembly 41 actuator means 50 acts upon the metering piston 46 stem by means of a rod 49 connected thereto. Such rod 49 moves piston 45 downwards so that a sufficient amount of product phase 12a is sucked into cylinder 45. During this operation, rotatable three-way valve 44 is in a position such that it connects hopper 43 to cylinder 45.

Once the piston 46 has reached its lowest point, corresponding to a sufficient amount of product phase 12a, the three-way valve 44 is rotated so as to communicate the metering piston 46 with the manifold 47 feeding the first filling nozzle 31.

Piston 46 is now driven upwards thus forcing product phase 12a contained in the cylinder 45 along manifold 47 to the first nozzle 31.

Simultaneously, the opening of the first nozzle 31 shutter 33 takes place along with the outflow of product phase 12a from nozzle 31 orifice until termination of the same.

The same operation also applies to the further filling assemblies 42, each time delayed by a preset number of machine degrees so as to obtain a time offset of the opening and closing steps of nozzles 31,32.

In practice, it has been noted that the multi-phase device according to the invention thus described is able to offer a different effect depending on the overall dispensed amount. In fact, the composition and so the visual, sensory or functional effects change depending on the number of supplies.

Indeed, it has been ascertained how the individual phases of the multi-phase device according to the invention can be dispensed in a progressively increasing and/or decreasing manner, offering an opportunity to make available for use a combination of products having different formulations (in particular cream, paste or gel), dyes, aromas, flavours, and/or active ingredients according to the particular supply.

It has been established moreover how it is possible to easily obtain a multi-phase product according to the invention by the filling method described.

Last but not least, it has been established how the filling equipment described is able to implement such method.

The invention thus conceived is susceptible to many modifications, additions and variations, all of which are within the scope of the inventive concept.

In practice, the materials used, as well as the dimensions may be any according to technical requirements. Moreover, all the details may be replaced with technically equivalent elements.

Hence, the scope of protection defined by the claims shall not be limited by the preferred embodiments exemplified in the description and in the figures, but it shall embrace all the novelty characteristics residing in the present invention, comprising those characteristics which would be deemed as equivalent by the person skilled in the art.

## Claims

1. Progressive effect multi-phase product (10) comprising a container (11) having a dispensing opening (13) and at least two product phases (12a,12b,12c), said at least two product phases (12a,12b,12c) being dispensable from said container (11) and being arranged in said container (11) such as to identify a first container portion (I) substantially comprising only one first product phase (12a), at a first end of said container, and a second container portion (II) wherein said first product phase (12a) is substantially absent, at a second end of said container, said second portion (II) comprising at least one second product phase (12b,12c), **characterised in that** the arrangement in said container (11) of said at least two product phases (12a,12b,12c) is such as to further identify a container portion (III) intermediate said first (I) and second (II) portions comprising at least two longitudinally flanking product phases (12a, 12b, 12c), said at least two product phases (12a,12b,12c) comprising at least said first product phase (12a).

2. Multi-phase product (10) according to claim 1, **characterised in that** said intermediate portion (III) comprises two longitudinally flanking product phases (12a, 12b) following an oblique progression direction with respect to the main dimension of said container (11).

3. Multi-phase product (10) according to claim 1, **characterised in that** said intermediate portion (III) comprises two longitudinally flanking product phases (12a,12b) parallel to the main dimension of said container (11).

4. Multi-phase product (10) according to claim 1, **characterised in that** said intermediate portion (III) comprises two product phases (12a,12b) placed longitudinally flanking each other such that said first product phase (12a) forms a cone embedded in said second product phase (12b).

5. Multi-phase product (10) according to claim 1, **characterised in that** said intermediate portion (III) comprises three longitudinally flanking product phases (12a,12b,12c) following an oblique progression with respect to the main dimension of said container (11).

6. Multi-phase product (10) according to anyone of the previous claims, **characterised in that** said container (11) first end is close to said opening (13).

7. Multi-phase product (10) according to anyone of the previous claims, **characterised in that** said at least two product phases (12a,12b,12c) are distinguished by at least one different element chosen from the group consisting of:
- active ingredient;
- colouring;
- aroma;
- flavour;
- formulation.

8. Multi-phase product (10) according to anyone of the previous claims, **characterised in that** at least one of said product phases (12a,12b,12c) comprises an active ingredient.

9. Multi-phase product (10) according to claim 8, **characterised in that** said at least one product phase (12a,12b,12c) comprising an active ingredient is said first product phase (12a).

10. Multi-phase product (10) according to anyone of the previous claims, **characterised in that** said product phases (12a,12b,12c) are edible.

11. Method for filling a multi-phase product (10) according to anyone of claims 1-10, the filling method comprising the steps of:
a) immersing a plurality of nozzles (31,32) in said container (11) until its lower dead point is reached;
b) opening a first shutter (33) of a first nozzle (31) containing said first product phase (12a);
c) opening one second shutter (34) of a second nozzle (32) containing a further product phase (12b,12c), after a first predetermined offset;
d) closing said first shutter (33) of said first nozzle (31), after a second predetermined offset.

12. Method for filling a multi-phase product (10) according to anyone of claims 1-10, the filling method comprising the steps of:
a) immersing a plurality of nozzles (31,32) in said container (11) until its lower dead point is reached;
b) opening at least one second shutter (34) of a second nozzle (32) containing at least one second product phase (12b,12c);
c) opening one first shutter (33) of a first nozzle (31) containing said first product phase (12a), after a first predetermined offset;
d) closing said at least one second shutter (34) of said second nozzle (32), after a second predetermined offset.

13. Filling method according to claim 11 or 12, **characterised in that** said shutter (33,34) opening steps b) and c) and closing step d) are carried out in a gradual manner.

14. Filling method according to one of claims 11, 12 or 13 further comprising the step of:
e) opening at least one further shutter of a further nozzle containing a further product phase (12c).

15. Filling method according to claim 14 further comprising the step of:
f) closing said at least one further shutter.

16. Filling method according to claim 14 or 15, wherein said opening step e) is carried out in an offset manner with respect to said shutter opening steps b) and c).

17. Filling method according to one of claims 14-16, wherein said closing step is carried out in an offset manner with respect to said shutter closing step d).

18. Equipment for filling a multi-phase product (10) according to anyone of claims 1-10, the filling equipment consisting of at least two filling assemblies (41,42) each comprising a hopper (43) connected through a three-way valve (44) in an open position to a metering cylinder (45) comprising a piston (46), said three-way valve (44) connecting in a closed position said metering cylinder (45) to a manifold (47,48) for feeding a nozzle (31,32), **characterised in that** it comprises actuator means (50) adapted to drive said at least two filling assemblies (41,42) in a manner mutually offset over time.

19. Filling equipment according to claim 18, **characterised in that** said actuator means (50) is a mechanical cam or an electronic cam.

20. Filling equipment according to claim 18, **characterised in that** said electronic cam is a brushless motor.

## Patentansprüche

1. Progressiv wirkendes Mehrphasenprodukt (10), das einen Behälter (11) mit einer Ausgabeöffnung (13) und mindestens zwei Produktphasen (12a,12b,12c) umfasst, wobei diese mindestens zwei Produktphasen (12a,12b,12c) aus diesem Behälter (11) ausgegeben werden können und derart in diesem Behälter (11) angeordnet sind, dass an einem ersten Ende dieses Behälters ein erster Behälterbereich (I), der im Wesentlichen nur eine erste Produktphase (12a) enthält, und an einem zweiten Ende dieses Behälters ein zweiter Behälterbereich (II), in dem die erste Produktphase (12a) im Wesentlichen nicht vorhanden ist, zu identifizieren sind, wobei dieser zweite Bereich (II) mindestens eine zweite Produktphase (12b,12c) enthält, **dadurch gekennzeichnet, dass** die Anordnung der mindestens zwei Produktphasen (12a,12b,12c) in diesem Behälter (11) derart ist, dass außerdem ein dritter Behälterbereich (III) zwischen dem ersten (I) und dem zweiten (II) Bereich zu identifizieren ist, der mindestens zwei in Längsrichtung aneinander angrenzende Produktphasen (12a, 12b, 12c) enthält, wobei diese mindestens zwei Produktphasen (12a,12b,12c) mindestens die erste Produktphase (12a) umfassen.

2. Mehrphasenprodukt (10) nach Anspruch 1, **dadurch gekennzeichnet, dass** der Zwischenbereich (III) zwei in Längsrichtung aneinander angrenzende Produktphasen (12a, 12b) enthält, die einer zur Hauptabmessung des Behälters (11) schrägen Verlaufsrichtung folgen.

3. Mehrphasenprodukt (10) nach Anspruch 1, **dadurch gekennzeichnet, dass** der Zwischenbereich (III) zwei in Längsrichtung aneinander angrenzende Produktphasen (12a,12b) enthält, die parallel zur Hauptabmessung des Behälters (11) sind.

4. Mehrphasenprodukt (10) nach Anspruch 1, **dadurch gekennzeichnet, dass** der Zwischenbereich (III) zwei Produktphasen (12a,12b) enthält, die so in Längsrichtung aneinander angrenzend angeordnet sind, dass die erste Produktphase (12a) einen in die zweite Produktphase (12b) eingebetteten Kegel bildet.

5. Mehrphasenprodukt (10) nach Anspruch 1, **dadurch gekennzeichnet, dass** der Zwischenbereich (III) drei in Längsrichtung aneinander angrenzende Produktphasen (12a,12b,12c) enthält, die einem zur Hauptabmessung des Behälters (11) schrägen Verlauf folgen.

6. Mehrphasenprodukt (10) nach einem der vorherigen Ansprüche, **dadurch gekennzeichnet, dass** das erste Ende des Behälters (11) nahe der Öffnung (13) ist.

7. Mehrphasenprodukt (10) nach einem der vorherigen Ansprüche, **dadurch gekennzeichnet, dass** sich mindestens zwei Produktphasen (12a,12b,12c) durch mindestens ein verschiedenes Element unterscheiden, das aus der Gruppe ausgewählt ist, die aus Folgendem besteht:
- Wirkstoff;
- Farbton;
- Aroma;
- Geschmack;
- Formulierung.

8. Mehrphasenprodukt (10) nach einem der vorherigen Ansprüche, **dadurch gekennzeichnet, dass** mindestens eine der Produktphasen (12a,12b,12c) einen Wirkstoff umfasst.

9. Mehrphasenprodukt (10) nach Anspruch 8, **dadurch gekennzeichnet, dass** mindestens eine einen Wirkstoff enthaltende Produktphase (12a,12b,12c) die erste Produktphase (12a) ist.

10. Mehrphasenprodukt (10) nach einem der vorherigen Ansprüche, **dadurch gekennzeichnet, dass** die Produktphasen (12a, 12b, 12c) zum menschlichen Verzehr geeignet sind.

11. Verfahren zum Füllen eines Mehrphasenprodukts (10) nach einem der Ansprüche 1 bis 10, wobei das Füllverfahren die folgenden Schritte umfasst:
a) Eintauchen einer Vielzahl von Düsen (31,32) in den Behälter (11), bis sein unterer Totpunkt erreicht ist;
b) Öffnen eines ersten Verschlusses (33) einer ersten Düse (31), welche die erste Produktphase (12a) enthält;
c) Öffnen eines zweiten Verschlusses (34) einer zweiten Düse (32), die eine weitere Produktphase (12b,12c) enthält, nach einem ersten vorbestimmten Versatz;
d) Schließen des ersten Verschlusses (33) der ersten Düse (31) nach einem zweiten vorbestimmten Versatz.

12. Verfahren zum Füllen eines Mehrphasenprodukts (10) nach einem der Ansprüche 1 bis 10, wobei das Füllverfahren die folgenden Schritte umfasst:
a) Eintauchen einer Vielzahl von Düsen (31,32) in den Behälter (11), bis sein unterer Totpunkt erreicht ist;
b) Öffnen von mindestens einem zweiten Verschluss (34) einer zweiten Düse (32), die mindestens eine zweite Produktphase (12b,12c) enthält;
c) Öffnen eines ersten Verschlusses (33) einer ersten Düse (31), welche die erste Produktphase (12a) enthält, nach einem ersten vorbestimmten Versatz;
d) Schließen des mindestens einen zweiten Verschlusses (34) der zweiten Düse (32) nach einem zweiten vorbestimmten Versatz.

13. Füllverfahren nach Anspruch 11 oder 12, **dadurch gekennzeichnet, dass** die Schritte b) und c) des Öffnens und der Schritt d) des Schließens des Verschlusses (33,34) schrittweise ausgeführt werden.

14. Füllverfahren nach einem der Ansprüche 11, 12 oder 13, das ferner die folgenden Schritte umfasst:
e) Öffnen von mindestens einem weiteren Verschluss einer weiteren Düse, die eine weitere Produktphase (12c) enthält.

15. Füllverfahren nach Anspruch 14, das ferner den folgenden Schritt umfasst:
f) Schließen des mindestens einen weiteren Verschlusses.

16. Füllverfahren nach Anspruch 14 oder 15, bei dem der Öffnungsschritt e) versetzt zu den Verschlussöffnungsschritten b) und c) ausgeführt wird.

17. Füllverfahren nach einem der Ansprüche 14 bis 16, bei dem der Schließschritt versetzt zum Verschlussschließschritt d) ausgeführt wird.

18. Einrichtung zum Füllen eines Mehrphasenprodukts (10) nach einem der Ansprüche 1 bis 10, wobei die Fülleinrichtung aus mindestens zwei Füllanordnungen (41,42) besteht, die jeweils einen Trichter (43) umfassen, der durch ein Drei-Wege-Ventil (44) in einer geöffneten Stellung mit einem Dosierzylinder (45) verbunden ist, der einen Kolben (46) umfasst, wobei das Drei-Wege-Ventil (44) diesen Dosierzylinder (45) in einer geschlossenen Stellung mit einem Verteiler (47,48) zum Speisen einer Düse (31,32) verbindet, **dadurch gekennzeichnet, dass** sie ein Aktuatormittel (50) umfasst, das geeignet ist, die mindestens zwei Füllanordnungen (41,42) zeitlich zueinander versetzt anzutreiben.

19. Fülleinrichtung nach Anspruch 18, **dadurch gekennzeichnet, dass** das Aktuatormittel (50) ein mechanischer Nocken oder ein elektronischer Nocken ist.

20. Fülleinrichtung nach Anspruch 18, **dadurch gekennzeichnet, dass** der elektronische Nocken ein bürstenloser Motor ist.

## Revendications

1. Produit à phases multiples à effet progressif (10) comprenant un contenant (11) ayant une ouverture de distribution (13) et au moins deux phases de produit (12a, 12b, 12c), lesdites au moins deux phases de produit (12a, 12b, 12c) pouvant être distribuées à partir dudit contenant (11) et étant disposées dans ledit contenant (11) de telle manière à identifier une première partie de contenant (I) comprenant substantiellement uniquement une première phase de produit (12a), à une première extrémité dudit contenant, et une seconde partie de contenant (II) où ladite première phase de produit (12a) est substantiellement absente, à une seconde extrémité dudit contenant, ladite seconde partie (II) comprenant au moins une seconde phase de produit (12b, 12c), **caractérisé en ce que** la disposition dans ledit contenant (11) desdites au moins deux phases de produit (12a, 12b, 12c) est telle à identifier ultérieurement une partie de contenant (III) intermédiaire, lesdites première (I) et seconde (II) parties comprenant au moins deux phases de produit se flanquant longitudinalement (12a, 12b, 12c), lesdites au moins deux phases de produit (12a, 12b, 12c) comprenant au moins ladite première phase de produit (12a).

2. Produit à phases multiples (10) selon la revendication 1, **caractérisé en ce que** ladite partie intermédiaire (III) comprend deux phases de produit se flanquant longitudinalement (12a, 12b) suivant une direction de progression oblique par rapport à la dimension principale dudit contenant (11).

3. Produit à phases multiples (10) selon la revendication 1, **caractérisé en ce que** ladite partie intermédiaire (III) comprend deux phases de produit se flanquant longitudinalement (12a, 12b) parallèles à la dimension principale dudit contenant (11).

4. Produit à phases multiples (10) selon la revendication 1, **caractérisé en ce que** ladite partie intermédiaire (III) comprend deux phases de produit (12a, 12b) placées longitudinalement se flanquant l'une l'autre de telle sorte que ladite première phase de produit (12a) forme un cône intégré à ladite seconde phase de produit (12b).

5. Produit à phases multiples (10) selon la revendication 1, **caractérisé en ce que** ladite partie intermédiaire (III) comprend trois phases de produit se flanquant longitudinalement (12a, 12b, 12c) suivant une progression oblique par rapport à la dimension principale dudit contenant (11).

6. Produit à phases multiples (10) selon une quelconque des revendications précédentes, **caractérisé en ce que** ladite première extrémité du contenant (11) est proche de ladite ouverture (13).

7. Produit à phases multiples (10) selon une quelconque des revendications précédentes, **caractérisé en ce que** lesdites au moins deux phases de produit (12a, 12b, 12c) se distinguent par au moins un élément différent choisi à partir du groupe consistant eu
- principe actif ;
- colorant ;
- arôme ;
- goût ;
- formulation.

8. Produit à phases multiples (10) selon une quelconque des revendications précédentes, **caractérisé en ce qu'**au moins une desdites phases de produit (12a, 12b, 12c) comprend un principe actif.

9. Produit à phases multiples (10) selon la revendication 8, **caractérisé en ce que** ladite au moins une phase de produit (12a, 12b, 12c) comprenant un principe actif est ladite première phase de produit (12a).

10. Produit à phases multiples (10) selon une quelconque des revendications précédentes, **caractérisé en ce que** lesdites phases de produit (12a, 12b, 12c) sont comestibles.

11. Procédé de remplissage d'un produit à phases multiples (10) selon une quelconque des revendications 1-10, le procédé de remplissage comprenant les étapes consistant à :
a) immerger une pluralité de buses (31, 32) dans ledit contenant (11) jusqu'à ce que son point mort inférieur soit atteint ;
b) ouvrir un premier obturateur (33) d'une première buse (31) contenant ladite première phase de produit (12a) ;
c) ouvrir un second obturateur (34) d'une seconde buse (32) contenant une ultérieure phase de produit (12b, 12c), après un premier décalage prédéterminé ;
d) fermer ledit premier obturateur (33) de ladite première buse (31), après un second décalage prédéterminé.

12. Procédé de remplissage d'un produit à phases multiples (10) selon une quelconque des revendications 1-10, le procédé de remplissage comprenant les étapes consistant à :
a) immerger une pluralité de buses (31, 32) dans ledit contenant (11) jusqu'à ce que son point mort inférieur soit atteint ;
b) ouvrir au moins un second obturateur (34) d'une seconde buse (32) contenant au moins une seconde phase de produit (12b, 12c) ;
c) ouvrir un premier obturateur (33) d'une première buse (31) contenant ladite première phase de produit (12a), après un premier décalage prédéterminé ;
d) fermer ledit au moins un second obturateur (34) de ladite seconde buse (32), après un second décalage prédéterminé.

13. Procédé de remplissage selon la revendication 11 ou 12, **caractérisé en ce que** lesdites phases d'ouverture b) et c) et phase de fermeture d) d'obturateur (33, 34) sont exécutées de manière graduelle.

14. Procédé de remplissage selon l'une des revendications 11, 12 ou 13 comprenant en outre l'étape consistant à :
e) ouvrir au moins un ultérieur obturateur d'une ultérieure buse contenant une ultérieure phase de produit (12c).

15. Procédé de remplissage selon la revendication 14 comprenant en outre l'étape consistant à :
f) fermer ledit au moins un ultérieur obturateur.

16. Procédé de remplissage selon la revendication 14 ou 15, où ladite étape d'ouverture e) est exécutée de manière décalée par rapport auxdites étapes d'ouverture d'obturateur b) et c).

17. Procédé de remplissage selon l'une des revendications 14-16, où ladite étape de fermeture est exécutée de manière décalée par rapport à ladite étape de fermeture d'obturateur d).

18. Equipment de remplissage d'un produit à phases multiples (10) selon une quelconque des revendications 1-10, l'équipement de remplissage consistant en au moins deux ensembles de remplissage (41, 42) chacun comprenant une trémie (43) reliée à travers un robinet à trois voies (44) dans une position ouverte à un cylindre de dosage (45) comprenant un piston (46), ledit robinet à trois voies (44) reliant dans une position fermée ledit cylindre de dosage (45) à un collecteur (47, 48) pour alimenter une buse (31, 32), **caractérisé en ce qu'**il comprend un moyen d'actionnement (50) adapté pour entraîner lesdits au moins deux ensembles de remplissage (41, 42) de manière réciproquement décalée au fil du temps.

19. Equipement de remplissage selon la revendication 18, **caractérisé en ce que** ledit moyen d'actionnement (50) est une came mécanique ou une came électronique.

20. Equipement de remplissage selon la revendication 18, **caractérisé en ce que** ladite came électronique est un moteur sans balai.
